# FASCICULE DE BREVET EUROPEEN

(11) **EP 4 275 625 B1**
(45) Date de publication et mention de la délivrance du brevet: **12.03.2025**
(21) Numéro de dépôt: 23198217.4
(22) Date de dépôt: 13.08.2018
(51) Int. Cl.: A61C 1/00, A61B 17/16

(54) **SYSTÈME DE COMMANDE AVEC BOUTON STÉRILISABLE AMOVIBLE**
STEUERUNGSSYSTEM MIT ENTFERNBAREM STERILISIERBAREM KNOPF
CONTROL SYSTEM WITH REMOVABLE STERILIZABLE BUTTON

(43) Date de publication de la demande: 15.11.2023
(62) Demande divisionnaire de: 18188657.3
(73) Titulaire: Bien-Air Holding SA, 2504 Bienne (CH)
(72) Inventeur: BRASEY, Yvan, 1544 Gletterens (CH); ZILL, Corentin, 2608 Courtelary (CH); SARCHI, Davide, 8008 Zürich (CH)
(74) Mandataire: BOVARD AG

(56) Documents cités:
- WO-A1-2014/060676
- CN-A- 101 444 631
- US-A1- 2004 125 437
- US-A1- 2015 150 646

## Description

### Domaine technique de l'invention

La présente invention se rapporte au domaine des instruments et appareils pour les praticiens dans le secteur médical, tels que les dentistes et les chirurgiens dentaires. Plus précisément, elle concerne un appareil de commande pour le moteur de tels appareils.

### État de la technique

Les dentistes, hygiénistes et chirurgiens dentaires, ainsi que d'autres praticiens du secteur notamment de l'endodontie, parodontologie ou de la chirurgie orale ou de l'implantologie ont souvent recours à divers instruments et appareils dotés d'un outil rotatif actionnés par un moteur (micromoteur) électrique. Ces moteurs sont dotés d'une puissance prédéfinie, qui est adaptée aux opérations à réaliser. Ainsi par exemple selon les applications (nettoyage, polissage, traitement de racine, etc.), les opérations de meulage ou de fraisage nécessitent l'application de différentes vitesses et différents couples adaptés à la force de contact exercée sur l'outil permettent de gérer efficacement le niveau d'abrasion ou de perçage sans générer de surchauffe excessive de l'outil qui pourrait abîmer les tissus environnants.

Pour contrôler les micromoteurs de tels outils, différentes unités de commande sont disponibles sur le marché. La station de commande *Optima* de la demanderesse permet par exemple d'effectuer des interventions prophylactiques ou restauratrices en fournissant une alternative aux turbines, habituellement employées pour ce type d'opérations, mais qui présente l'inconvénient d'être beaucoup plus bruyantes en raison de la vitesse de rotation très élevée (supérieure à 100000 tours par minute) de l'outil entraîné par des moyens pneumatiques. Le contrôle de la vitesse y est effectué à l'aide d'une roulette centrale disposée sur la face avant de la console, prévue spécifiquement et uniquement à cet effet.

Dans le domaine de l'implantologie et de la chirurgie maxillo-faciale, on connaît par ailleurs d'autres types d'unités de commande comme la console *Chiropro* de la demanderesse, qui est pourvue d'un écran de contrôle ainsi que de plusieurs touches de commande agencées sur un clavier substantiellement plat. Les différents modes de fonctionnement y sont sélectionnés notamment à l'aide de flèches et actionnés à l'aide d'une touche centrale de validation. Un premier inconvénient de ce type de console est lié au caractère relativement peu ergonomique de l'interface de commande, nécessitant souvent des pressions multiples successives sur les différents boutons pour changer de programme. Un deuxième inconvénient de ce type d'unité de commande est lié au processus de désinfection et décontamination du clavier de la console, qui lors de chaque opération, est susceptible d'être intégralement contaminé suite aux différentes manipulations effectuées par le chirurgien. Un tel processus s'avère relativement fastidieux parce qu'il est impossible de stériliser l'ensemble de la console, en la réchauffant à 135°C, selon les procédures recommandées, sans l'endommager sérieusement. Il est donc nécessaire de se limiter à une désinfection manuelle avec des produits chimiques, souvent agressifs pour les matériaux de la console et donc produisant une dégradation esthétique sur le moyen ou long terme.

US 2015/150646 A1 et WO 2014/060676 A1 divulguent des systèmes de commande pour micromoteur comportant des boutons amovibles.

Il existe donc un besoin pour des solutions d'interfaces de commande dépourvues des inconvénients mentionnés ci-dessus.

### Résumé de l'invention

L'invention a pour but de fournir un système de commande pourvu d'une interface simple, pratique d'utilisation, et facile à stériliser en cas de besoin.

Selon l'invention, ce but est atteint grâce à un système de commande pour micromoteur à usage dentaire ou chirurgical comprenant un boîtier de commande pourvu d'une molette de réglage montée rotative autour d'un axe de rotation, caractérisé en ce que la molette de réglage est formée d'un support de commande rotatif autour dudit axe de rotation et solidaire d'un encodeur intégré au boîtier, et d'un bouton stérilisable couplé de façon amovible au support de commande, le couplage étant réalisé par friction, par clipsage, ou magnétiquement. Grâce à cet agencement de la molette de réglage, il est possible de monter et démonter aisément la partie manipulée par le chirurgien et stériliser cette dernière indépendamment du reste du boîtier de commande. Par ailleurs, la partie stérilisable peut désormais être considérée comme une pièce d'usure pouvant être remplacée de façon modulaire indépendamment du reste du système de commande, et cette dernière peut par conséquent être stérilisée et lavée par des produits chimiques plus agressifs plus efficaces.

Le couplage réalisé par friction, par clipsage, ou magnétiquement permet un montage et un démontage manuel du bouton stérilisable amovible, c'est-à-dire sans l'aide d'aucun outil auxiliaire. La commodité d'usage est ainsi grandement améliorée pour ce type d'opérations d'assemblage et de remplacement de la partie stérilisable amovible.

Selon un mode de réalisation préférentiel, la molette de réglage est prévue à la fois pour la sélection en rotation et pour l'actionnement par pression de fonctions, lesquelles sont visualisables sur un écran de contrôle du boîtier, ce qui permet de renforcer le niveau d'ergonomie par rapport aux produits existants, dans lesquels ces deux fonctionnalités étaient jusqu'à présent réalisées à l'aide d'éléments d'actionnement dissociés.

Selon un mode de réalisation préférentiel, le bouton stérilisable amovible est terminé, au niveau de son extrémité inférieure, par une collerette s'étendant radialement vers l'extérieur, ce qui présente l'avantage, d'une part, d'éviter toute contamination intempestive du boîtier lors de la manipulation de la molette de réglage, et d'autre part, de faciliter le démontage du bouton stérilisable amovible en augmentant le diamètre de l'objet au niveau de la zone de saisie et en facilite ainsi la préhension manuelle. Par ailleurs, dans le cas où des pressions sont nécessaires sur la molette, celles-ci peuvent également être exécutées plus aisément en appuyant les doigts sur la surface extérieure de la collerette.

Selon un mode de réalisation préférentiel, le bouton stérilisable amovible est de préférence couplé au support de commande de manière à ce que, en position assemblée, un jeu de fonctionnement d'au moins 0.2mm subsiste axialement entre l'extrémité inférieure du bouton stérilisable et la surface externe du boîtier lorsque celui-ci se trouve au repos, c'est-à-dire en position non enfoncée. Ainsi, les doigts peuvent passer légèrement derrière le bouton pour le saisir et l'extraire aisément du boîtier lors d'une opération de démontage.

Selon un mode de réalisation préférentiel, le bouton stérilisable amovible présente une cavité centrale pourvue d'au moins une première surface de contact intérieure pour l'accouplement à une surface d'appui et des moyens de retenue axiaux et d'entraînement en rotation agencés de part et d'autre du bouton stérilisable amovible et du support de commande. Un tel agencement permet d'amener simplement le bouton stérilisable amovible en butée contre le support de commande lors du montage, tout en garantissant une bonne qualité fonctionnelle de la molette qui peut ainsi transmettre efficacement le couple d'actionnement et éviter parallèlement tout risque d'arrachement involontaire du bouton stérilisable amovible. Par ailleurs, le mécanisme de couplage est caché sous le capot du bouton, ce qui permet de préserver au mieux l'esthétique du dispositif de commande malgré les améliorations opérationnelles conférées.

De préférence, la cavité centrale du bouton stérilisable amovible débouche sur un épaulement périphérique intérieur. Un tel aménagement vise à assurer la régularité de la qualité de l'accouplement entre bouton stérilisable amovible au support de commande indépendamment des cotes de fabrication, qui peuvent légèrement fluctuer pour chacune de ces pièces en fonction des tolérances d'assemblage. La qualité de cet accouplement permet notamment d'assurer une bonne transmission systématique du couple à l'encodeur.

Selon un mode de réalisation particulièrement préférentiel, le bouton stérilisable amovible est couplé magnétiquement au support de commande, une pièce magnétique active étant intégrée dans le support et une pièce magnétique passive étant intégrée dans le bouton stérilisable amovible, l'intensité de la force magnétique exercée étant comprise entre 5 et 15 Newton. Un tel agencement permet de conférer un excellent compromis entre la qualité de la tenue du bouton sur son support et la facilité des opérations de montage/démontage.

Selon une variante préférentielle liée à un tel mode de couplage magnétique, le bouton stérilisable amovible comprend une cavité centrale, comme selon un mode de réalisation préférentiel précédemment décrit, mais qui contient par ailleurs une deuxième surface de contact intérieure conique, amenée en appui, en position assemblée de la molette de réglage, contre une surface d'accouplement latérale du support de commande, et ces surfaces présentent une inclinaison de 15 degrés par rapport à l'axe de rotation (A-A) de la molette de réglage. Un tel agencement pour le couplage permet une construction très simple d'usinage ne nécessitant aucune surface ou élément d'accrochage ni d'éléments d'entraînement en rotation car l'orientation des lignes de champ magnétique permet une transmission optimale du couple.

Selon une autre variante préférentielle toujours liée au mode de couplage magnétique, la pièce magnétique active intégrée dans le support de commande est un premier aimant permanent agencé de manière à présenter une magnétisation orientée perpendiculairement par rapport à l'axe de rotation de la molette de réglage, et un deuxième aimant permanent est intégré dans le bouton stérilisable amovible de manière à présenter une magnétisation orientée parallèlement au premier aimant permanent. Dans une telle configuration, non seulement une force d'attirance magnétique s'établira entre le bouton et le support, mais un couple magnétique entre le bouton et le support exercé via les deux aimants permanents orientera naturellement le bouton sur le support lors de sa mise en place, ce qui positionnera ce dernier dans une position angulaire indexée prédéfinie, d'une part, et d'autre part, permettra d'entrainer encore plus efficacement en rotation le support lors de la rotation manuelle du bouton en raison de la tendance naturelle de préservation de l'alignement des pôles des deux aimants.

Selon encore une autre variante préférentielle liée au mode de couplage magnétique, la pièce magnétique active intégrée dans le support de commande est un premier aimant permanent agencé de manière à présenter une magnétisation orientée cette fois directement selon l'axe de rotation (A-A) de la molette de réglage, et un deuxième aimant permanent est intégré dans le bouton stérilisable amovible de manière à présenter une magnétisation orientée également selon l'axe de rotation (A-A) de la molette de réglage, mais dans le sens opposé par rapport à celui de ce premier aimant permanent. Une telle configuration d'aimants en opposition de polarité l'un face à l'autre permet d'établir une force de répulsion entre le bouton et le support quand ils sont distants l'un de l'autre, et d'assurer simultanément une force d'attirance magnétique importante quand le bouton est amené en proximité du support, et ce grâce à la déviation des lignes de champ magnétique provoquée par le composant ferromagnétique (c'est-à-dire la pièce magnétique passive) intégré(e) dans le capot du bouton. Ainsi lors de la phase de positionnement du bouton, l'utilisateur ressentira initialement une résistance et ensuite un effet de clipsage automatique du bouton sur le support, simulant donc le ressenti d'un clipsage mécanique. Lors de la phase de démontage du bouton, il suffira au contraire à l'utilisateur d'éloigner légèrement le bouton ou de l'incliner par rapport à l'axe du support pour profiter d'une force de répulsion magnétique favorisant son extraction, ce qui permettra d'éviter encore plus efficacement tout contact involontaire entre le bouton, contaminé, et l'écran ou toute autre partie du boîtier de commande.

Selon un mode de réalisation alternatif, le bouton stérilisable amovible peut être couplé par friction au support de commande grâce à des joints de type O-ring insérés dans des gorges latérales d'accouplement aménagées indifféremment sur le bouton stérilisable amovible ou le support de commande de la molette de réglage. Un avantage de la solution conférée est de pouvoir utiliser les mêmes éléments à la fois pour la retenue axiale et l'entraînement en rotation, c'est-à-dire la transmission du couple. Néanmoins, par rapport au mode de couplage magnétique, la durée de vie est sensiblement réduite et les O-rings devront être régulièrement changés pour assurer la qualité du couplage.

Selon un autre mode de réalisation, le bouton stérilisable amovible peut être assemblé au support de commande à l'aide de clips déformables élastiquement, et qui sont pourvus d'ergots d'accouplement destinés à être insérés dans des rainures d'accrochage. Un tel agencement permet également d'utiliser les mêmes éléments à la fois pour la retenue axiale et pour l'entraînement en rotation; une telle solution est toutefois sujette au fluage qui ne permet pas d'assurer durablement la qualité du couplage.

La présente invention concerne par ailleurs un boîtier de commande ainsi qu'un bouton stérilisable pris indépendamment l'un de l'autre, car ces deux produits peuvent être vendus séparément, même s'ils concernent le même système de commande proposé.

### Brève description des dessins

D'autres avantages et caractéristiques ressortiront plus clairement de la description qui va suivre de modes de réalisation pour la mise en œuvre de l'invention, donnés à titre d'exemple non limitatifs et illustrés sur les dessins annexés, parmi lesquels:
- la figure 1A est une vue en perspective schématique d'un système de commande selon un mode de réalisation préférentiel de l'invention, dans une configuration où la molette de réglage est susceptible d'être actionnée;
- la figure 1B est une vue en perspective schématique d'un système de commande selon un mode de réalisation préférentiel de l'invention, représentant le bouton stérilisable amovible de la molette en position démontée par rapport au boîtier de commande;
- la figure 2 est une vue en perspective de dessous d'un bouton stérilisable amovible selon un mode de réalisation préférentiel utilisant un mode de couplage magnétique par rapport à un support de commande;
- la figure 3 est une vue en coupe d'une molette de commande selon un mode de réalisation préférentiel utilisant un mode de couplage magnétique, représentant le bouton stérilisable amovible en position assemblée sur le support de commande du boîtier;
- la figure 4 illustre schématiquement un mode de réalisation alternatif pour un couplage magnétique utilisant deux aimants permanents agencés de part et d'autre du support de commande du boîtier et du bouton stérilisable amovible, selon une direction de magnétisation orthogonale à l'axe de rotation de la molette de réglage;
- la figure 5 illustre schématiquement un autre mode de réalisation alternatif pour un couplage magnétique utilisant deux aimants permanents agencés de part et d'autre du support de commande du boîtier et du bouton stérilisable amovible, cette fois avec une direction de magnétisation orientée selon l'axe de rotation de la molette de réglage, et en opposition de polarité;
- la figure 6 illustre une vue en coupe d'un mode de réalisation alternatif pour l'invention utilisant un mode de couplage par friction entre le bouton stérilisable amovible et le support de commande du boîtier;
- la figure 7 illustre une vue en coupe d'un mode de réalisation alternatif pour l'invention utilisant un mode de couplage selon lequel le bouton stérilisable amovible est clipsé au support de commande du boîtier, avec une vue en perspective de dessous d'un bouton stérilisable associé mettant en évidence les clips de fixation servant parallèlement à l'entraînement en rotation de la molette.

### Description de modes de réalisation préférentiels de l'invention

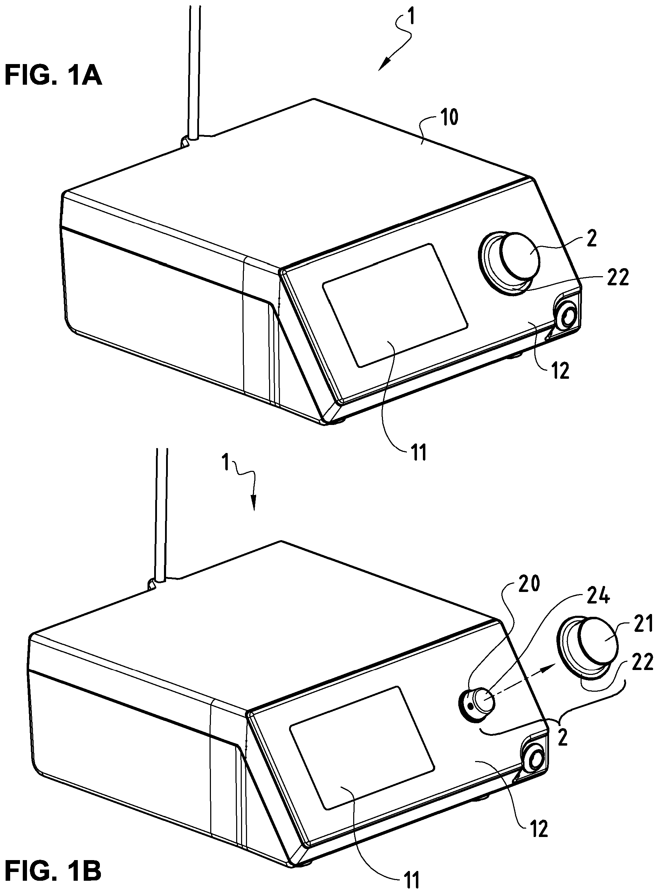

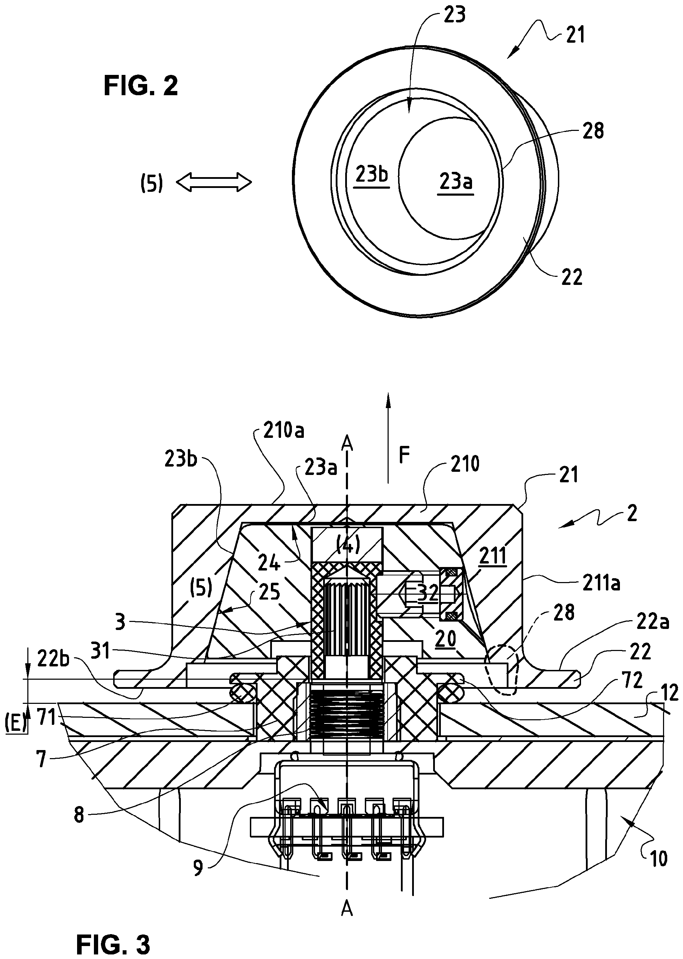

Les figures 1A et 1B représentent des vues en perspective d'un système de commande 1 pourvu d'un bouton stérilisable 21 amovible selon un mode de réalisation préférentiel pour la présente invention, utilisant un mode de couplage magnétique. Le système de commande 1 utilise un boîtier 10 de commande qui comprend classiquement un écran de contrôle 11 prévu au niveau de la surface externe 12 du boîtier de commande 10, ainsi que d'une molette de réglage 2, pourvue d'un encodeur - non visible sur ces figures mais qui est représenté plus loin notamment sur la figure 3 - et avec laquelle il est possible de sélectionner des modes ou des fonctions prédéfinies en la faisant tourner dans un sens ou dans l'autre. Ensuite, pour valider ou respectivement activer les modes ou les fonctions, une légère pression peut être exercée sur la molette 2. Ainsi, le niveau d'ergonomie est significativement amélioré par rapport aux systèmes de commande utilisant des touches dédiées ou n'importe quels éléments dissociés pour la sélection et l'activation de modes ou de fonctions.

Sur la figure 1A illustrant le système de commande 1 en position assemblée, la molette de réglage 2 ne semble pas différer d'une molette usuelle solidaire du boîtier 10 de commande et fixée à demeure sur celui-ci; on peut également distinguer une collerette 22 terminant la molette 2, ou plus exactement le bouton stérilisable 21 amovible de celle-ci, du côté du boîtier. Cette collerette 22 s'étend radialement vers l'extérieur, ce qui permet de faciliter sa préhension lors d'opérations de sélection et d'activation tout en protégeant la surface extérieure 12 du boîtier de commande 10 d'un contact non souhaité avec les doigts. Par ailleurs, l'opération de démontage de la partie amovible de la molette de réglage 2 est également facilitée grâce à la légère augmentation de diamètre qui permet de faire passer plus facilement les doigts juste sous le bouton stérilisable 21 amovible pour le saisir en vue de le détacher du boîtier 10 de commande.

La figure 1B illustre par contre la nature amovible du couplage entre le bouton stérilisable 21 et un support de commande 20 solidaire du boîtier 10. Le support de commande 20 est monté rotatif vis-à-vis du boîtier de commande du système de commande 1, et constitue une première partie de la molette de réglage 2; l'autre partie étant formée par un bouton stérilisable 21 sur lequel est formée la collerette 22 s'étendant radialement vers l'extérieur à sa base. Cet agencement permet ainsi de stériliser plus efficacement, et de façon modulaire, la partie qui est a priori la seule qui doit être manipulée et ainsi la seule susceptible de requérir un tel traitement après chaque utilisation.

La figure 2 montre le bouton stérilisable 21 amovible pris isolément du reste du système de commande 1, et qui peut ainsi également être vendu séparément. Ce bouton stérilisable 21 est de préférence constitué au moins partiellement d'un matériau ferromagnétique tel que de l'acier magnétisable afin de constituer une pièce magnétique passive 5 destinée à coopérer avec une pièce magnétique active 4 telle qu'un aimant disposé dans le support de commande 20 de la molette de réglage 2. La forme du bouton stérilisable 21 amovible correspond sensiblement à celle d'un chapeau, avec une collerette 22 s'étendant radialement à sa base et laissant apparaître une cavité centrale 23 permettant l'insertion du support de commande 20. La cavité centrale 23 est refermée par un fond comprenant une première surface de contact intérieure 23a, de préférence plane, et destinée à être accouplée à une surface d'appui 24 du support de réglage 20, et possède une deuxième surface de contact intérieure 23b, de préférence conique, sur laquelle une surface d'accouplement latérale 25 du support de réglage 20 vient en appui. Un épaulement périphérique intérieur 28 est toutefois prévu pour aménager un espace supplémentaire à l'entrée de la cavité centrale 23.

La figure 3 explique le mode d'accouplement entre le support de réglage 20 et le bouton stérilisable amovible 21 selon un mode de réalisation préférentiel de l'invention, dans lequel le couplage est magnétique et les surfaces latérales à la fois du support de réglage 20 et du bouton stérilisable amovible sont coniques avec une inclinaison comprise entre 10 et 20 degrés, et de préférence 15 degrés, de manière à présenter une orientation optimale par rapport aux lignes de champ générées par l'aimant intégré dans le support de commande 20 de la molette de réglage 2 et dont la direction de magnétisation est ici orientée perpendiculairement à l'axe de rotation A-A de la molette de réglage 2. Selon ce mode de réalisation préférentiel, on retrouve donc une pièce magnétique active 4, c'est-à-dire l'aimant permanent intégré dans le support de commande 20, tandis que l'intégralité du bouton stérilisable amovible 21 constitue une pièce magnétique passive 5. La section du support de commande 20 est ici trapézoïdale, matérialisant un volume de forme conique tronqué à son sommet. La partie supérieure du trapèze correspond ainsi à la surface d'appui 24 du support plane, qui est amenée en butée contre la première surface de contact intérieure 23 du bouton stérilisable 21 amovible, constituant la face interne du capot 210. La force d'attraction magnétique constitue ainsi à elle seule des moyens de retenue axiaux pour maintenir ces deux surfaces en appui l'une contre l'autre. Ensuite, l'inclinaison de la deuxième surface de contact intérieure 23b et de la surface d'accouplement latérale 25 permet, lorsque le bouton est actionné en rotation par l'utilisateur, d'entraîner plus efficacement en rotation l'encodeur 3 intégré au support de commande 20, qu'avec une coopération de surfaces cylindriques, pour lesquels la transmission du couple n'est pas optimale. L'encodeur 3 est formé ici par une tige filetée 31 montée rotative selon l'axe A-A de rotation de la molette de réglage 2 par rapport au boîtier de commande 10, et d'une vis 32 montée perpendiculairement à cet axe de rotation A-A.

Sur la figure 3, on peut également distinguer le capot 210 formant le haut du bouton stérilisable amovible 21, et la jupe axiale 211 dont l'épaisseur latérale s'amincit au fur et à mesure que l'on descend vers le bas, jusqu'à atteindre la collerette 22 orientée radialement vers l'extérieur. La surface latérale externe 211a du bouton stérilisable 21 amovible est sensiblement cylindrique, afin de permettre une préhension facile de ce dernier pour un actionnement en rotation pour une sélection, typiquement par le biais d'un menu déroulant. Lorsque l'utilisateur doit exercer une pression pour valider une fonction ou changer de mode, il peut appuyer soit sur la surface supérieure du capot 210a, soit sur la surface supérieure de la collerette 22a; le support de commande 20 sera alors entraîné vers le bas en allant à l'encontre de la force de rappel F du ressort 8 orientée vers le haut pour réaliser un contact électrique de type résistif avec le circuit électronique de commande 9. Immédiatement après un tel actionnement, le support de commande 20 sera ramené dans sa position de repos sous l'action de la force de rappel F exercée par le ressort de rappel 8.

La molette de réglage 2 est montée dans un tube d'insertion 7 dans le boîtier, qui présente un épaulement supérieur 72 recouvrant partiellement la surface externe 12 du boîtier de commande 10, et qui est apposé sur un joint d'étanchéification 71. Néanmoins, si un tel agencement garantit d'excellentes propriétés d'étanchéité par rapport à l'intérieur du boîtier de commande 10, un volume additionnel est toutefois généré au-dessus de ce dernier, et qui ne devrait pas empêcher l'action d'une pression sur le bouton - transmis directement au support de commande 20 - jusqu'à obtenir un contact électrique avec le circuit de commande 9. C'est une des raisons pour lesquelles l'épaulement périphérique intérieur 28 (matérialisé par la partie hachurée) est prévu à l'embouchure de la cavité centrale 23 pour conférer un évidement additionnel évitant toute interférence potentielle avec l'épaulement supérieur 72 du tube d'insertion de la molette.

Toujours sur la figure 3, on peut constater que la molette de réglage 2 est en position enfoncée, tandis que la flèche matérialisée par la référence E définit un jeu de fonctionnement au repos, c'est-à-dire l'espacement entre la surface externe du boîtier 12 et la surface inférieure de la collerette 22b. Ce jeu de fonctionnement est défini comme étant égal de préférence à au moins 0.2 millimètres, et peut aller de préférence jusqu'à 1 millimètre pour permettre aisément la préhension du bouton stérilisable 21 amovible et son retrait après utilisation.

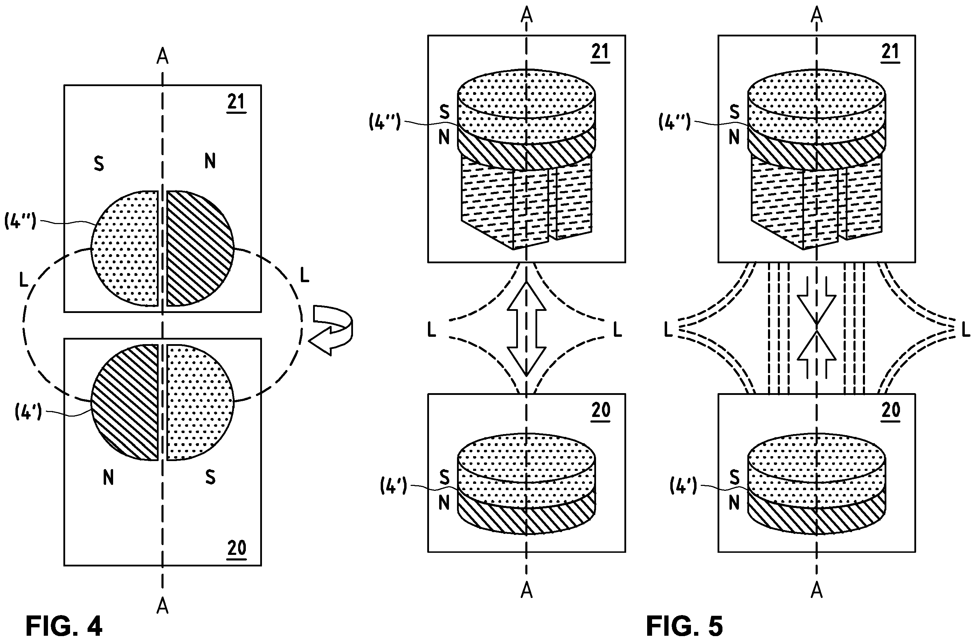

Selon le mode de réalisation préférentiel illustré sur les figures qui précèdent où le bouton stérilisable 21 amovible est couplé magnétiquement au support de commande 20, seule une pièce magnétique active 4 est intégrée dans le support de commande 20, tandis que le bouton stérilisable 21 amovible constitue une pièce magnétique passive 5 intégrale, de telle sorte que l'intensité de la force magnétique exercée soit comprise de préférence entre 5 et 15 Newton. Cette plage de valeurs relative à l'intensité de la force magnétique permet que les opérations de démontage demeurent aisées sans nécessiter de geste brusque ou violent pour retirer le bouton stérilisable 21 amovible. Néanmoins, d'autres variantes sont possibles en restant toujours dans le cadre d'un mode de couplage magnétique, en utilisant par exemple un bouton stérilisable 21 amovible composé uniquement partiellement de matériau magnétique, ou encore en agençant des aimants permanents de part et d'autre du support de commande 20 et du bouton stérilisable 21 amovible, comme expliqué à l'aide des schémas des figures 4 et 5 qui suivent.

La figure 4 illustre schématiquement une variante préférentielle pour un mode de couplage de type magnétique entre le support de commande 20 et le bouton stérilisable 21 amovible, selon laquelle, en plus d'un premier aimant permanent 4' constituant une pièce magnétique active 4 agencée similairement à celle de la figure 3, c'est-à-dire avec une direction de magnétisation orthogonale à l'axe de rotation A-A de la molette de réglage 2, on agence un deuxième aimant permanent 4" dans le bouton stérilisable 21 amovible selon une direction de magnétisation comprise dans un plan parallèle, superposé à celui du premier aimant permanent 4'. Ainsi, en position montée du bouton stérilisable 21 amovible sur le support de commande 20, les deux aimants permanents 4' et 4" auront tendance à s'orienter naturellement selon une direction de magnétisation parallèle l'une par rapport à l'autre, en raison de la préservation d'alignement des pôles suivant des lois de la physique. De cette manière, deux effets techniques avantageux sont réalisés, le premier étant d'obtenir une position d'indexation angulaire identique du bouton stérilisable 21 amovible suite à chaque montage sur son support de commande 20, et le deuxième étant de garantir encore une meilleure transmission du couple exercé sur le bouton stérilisable 21 amovible au support de commande 20 lors de l'actionnement en rotation de la molette 2 (matérialisée par la flèche dans le sens des aiguilles d'une montre sur la figure 4, montrant également la direction des lignes de champ L et les pôles Nord « N » et Sud « S » de chaque aimant). Cette variante de couplage présente l'avantage de dispenser, au moins partiellement, mais virtuellement intégralement, le bouton stérilisable 21 amovible de tout matériau ferromagnétique pour obtenir les effets recherchés en termes de qualité de transmission de couple et de tenue sur le support de commande 20; toutefois, on privilégiera ici encore, avec ou sans présence de matériau ferromagnétique passif, de préférence un agencement d'aimants permanents permettant d'obtenir une force d'attraction comprise dans les mêmes plages de valeurs que celles mentionnées précédemment, à savoir entre 5 et 15 Newton.

La figure 5 illustre encore une autre variante préférentielle utilisant un mode de couplage magnétique utilisant deux aimants permanents agencés respectivement de part et d'autre du support de commande 20 et du bouton stérilisable 21 amovible. Toutefois, contrairement à l'agencement de la figure 4 qui précède, le premier aimant permanent 4' du support de commande 20 et le deuxième aimant permanent 4" du bouton stérilisable amovible sont désormais agencés directement selon l'axe de rotation A-A de la molette de réglage, et en opposition de polarité (les pôles Nord « N » de chacun des aimants se faisant face l'un à l'autre). Ainsi, comme représenté sur la gauche de la figure 5, lorsque le bouton stérilisable 21 amovible est sensiblement éloigné du support de commande 20, les lignes de champ L de chacun des aimants sont dirigées les unes par rapport aux autres de telle sorte qu'un effet répulsif matérialisé par la flèche est généré entre ces deux pièces. Néanmoins, lorsque le bouton stérilisable 21 amovible est suffisamment rapproché du support de commande 20, comme illustré sur la partie droite de la figure 5, la force de répulsion magnétique se transforme en une force d'attraction magnétique, au vu de la réorganisation spatiale des lignes de champ L qui tend à maximiser le flux magnétique à travers les différents composants. Selon cette variante, l'opération de montage procure une sorte « d'effet clic » dès que l'on franchit le seuil de proximité mutuel entre bouton stérilisable 21 amovible et le support de commande 20 transformant la force de répulsion magnétique à une force d'attraction magnétique. En termes de transmission de couple et d'orientation préférentielle (indexation angulaire du bouton stérilisable 21 amovible par rapport au support de commande 20), on peut obtenir les mêmes avantages que ceux conférés par la variante précédente illustrée sur la figure 4; la variante de la figure 5 présente toutefois l'avantage additionnel de faciliter l'opération de démontage grâce à la force de répulsion magnétique résultante de l'éloignement et/ou de l'inclinaison du bouton stérilisable 21 amovible, qui favorise l'extraction de ce dernier.

L'avantage commun à toutes les variantes préférentielles utilisant un mode de couplage magnétique entre le bouton stérilisable 21 amovible et le support de commande 20 est que les opérations de montage et de démontage sont particulièrement simples, ne nécessitant aucun outil, et que ces opérations peuvent être répétées un très grand nombre de fois sans porter préjudice à la tenue du bouton stérilisable 21 amovible sur le support de commande 20 ni à la transmission du couple exercé par l'utilisateur à l'encodeur 3 de la molette de réglage 2. Même dans le cas où des aimants sont insérés dans le bouton stérilisable 21 amovible, il est possible, en utilisant par exemple des aimants fabriqués en Samarium Cobalt, de soumettre ces derniers à plusieurs centaines de cycles de stérilisation à 135°C ou plus sans altérer leurs propriétés magnétiques parce que leur température de Curie dépasse 300°C.

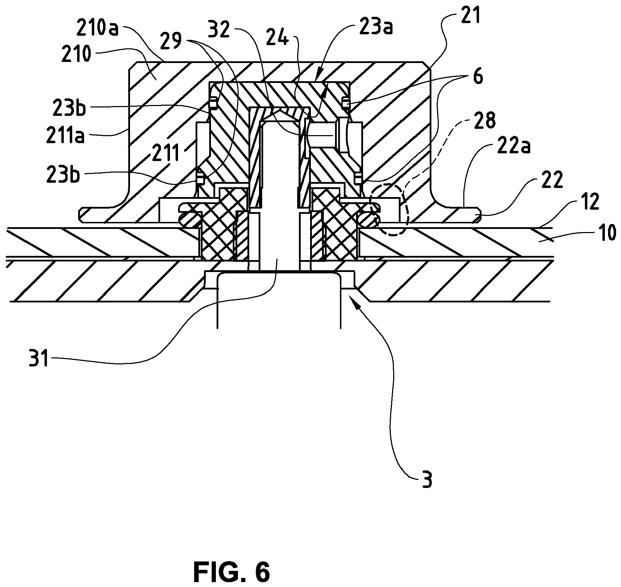

Selon une autre variante illustrée par la vue en coupe de la figure 6, le mode de couplage entre le bouton stérilisable 21 amovible et le support de commande 20 peut être réalisé par friction, par exemple à l'aide d'un ou plusieurs joints d'accouplement de type O-ring 6 insérés dans des gorges d'accouplement 29. Sur la figure 6, une première gorge d'accouplement 29 est disposée juste sous le capot 210, soit dans une portion assez haute du support de commande 20, et une deuxième gorge d'accouplement 29 est disposée au bas du support de commande, de telle sorte que les forces de frictions sont réparties sur deux joints O-ring 6, dont la compression contre des portions respectives de la deuxième surface de contact intérieure 23b du bouton stérilisable 21 amovible permet conjointement d'éviter tout arrachement de ce dernier et de transmettre le couple exercé sur lui à l'encodeur 3. Néanmoins, contrairement au mode de couplage magnétique, pour laquelle la durée de vie est excellente, cette solution nécessite un remplacement fréquent des joints pour garantir la qualité du couplage. Toutes les références de la figure 6 qui sont communes à celles de la figure 3 ne seront pas réexpliquées en détail; on pourra simplement constater que vu de l'extérieur, aucune différence n'est perceptible par rapport au mode de couplage magnétique; seules les formes des surfaces de contact intérieures du bouton stérilisable 21 amovible et la forme du support de commande 20 diffèrent éventuellement, mais la forme extérieure du bouton reste identique et les éléments servant au couplage demeurent cachés.

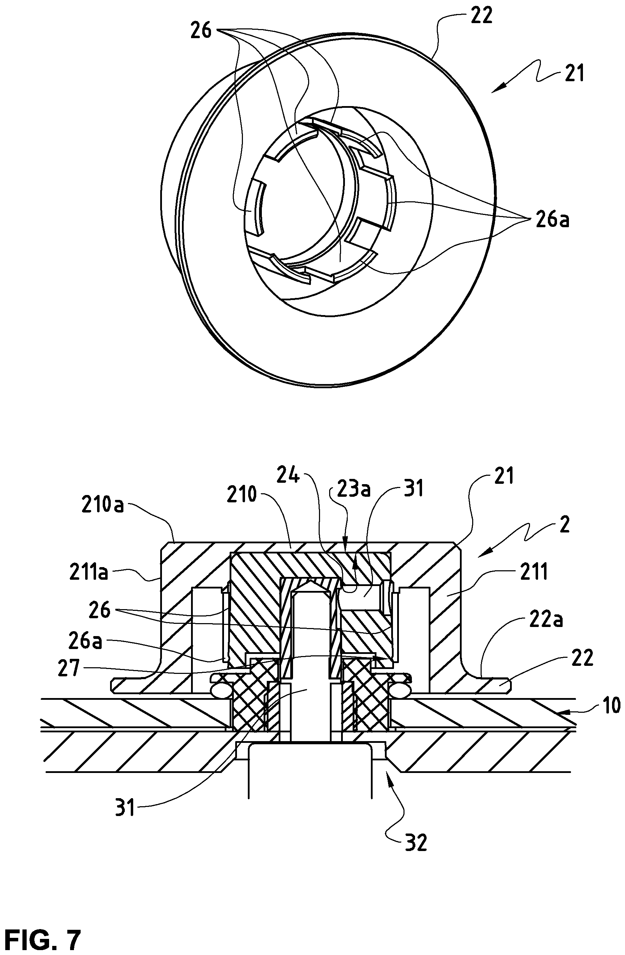

Selon encore une autre variante illustrée par la figure 7, le couplage peut être réalisé par clipsage à l'aide d'au moins un clip 26 déformable élastiquement (sur la figure 7, on peut en distinguer 6) et constitués par exemple d'un matériau plastique, présentant un ergot 26a à son extrémité, lequel est destiné à être inséré dans une rainure d'accrochage 27 disposée dans le support de commande 20. La coopération de l'ergot 26a dans la rainure d'accrochage 27 permet d'une part de garantir la retenue axiale du bouton stérilisable 21 amovible sur le support de commande 20 pour éviter tout arrachement, et également de garantir la transmission du couple. A cet effet, on pourra noter que la présence d'une jupe cylindrique faisant office de clip et qui serait insérée dans une gorge annulaire s'étendant sur l'intégralité du pourtour du support de commande 20 permettrait certes d'agir comme moyen de retenue axiale, mais ne permettrait pas de remplir cette deuxième fonction de transmission du couple, qui est au contraire d'autant mieux remplie que le nombre de clips est élevé et que les rainures d'accrochage 27 correspondantes sont profondes. L'agencement proposé et illustré notamment sur la figure 7 présente l'avantage d'utiliser les mêmes éléments à la fois pour la retenue axiale et la transmission de l'entraînement en rotation; elle présente néanmoins l'inconvénient d'être sujette au fluage des éléments plastiques utilisés pour les clips 6, et nécessiteraient par conséquent un remplacement fréquent de ces derniers pour garantir la qualité du couplage. Ici encore, toutes les références de la figure 6 qui sont communes à celles de la figure 3 ne seront pas réexpliquées en détail et aucune différence n'est perceptible vu de l'extérieur par rapport aux autres modes de couplage, la forme extérieure du bouton demeurant identique et les éléments impliqués pour le couplage étant cachés sous le capot 210 et la jupe axiale 211 du bouton stérilisable 21 amovible.

Dans ce qui précède, les modes de réalisation préférentiels et les variantes ont été données à titre d'exemple uniquement, sans avoir pour vocation de limiter la protection recherchée pour la présente invention. L'homme du métier comprendra que le système de commande 10 proposé pourrait posséder n'importe quelle constitution appropriée, potentiellement légèrement différente de celle décrite précédemment sans s'écarter de l'esprit de la présente invention ni réduire la portée conférée pour la présente invention.

En particulier, il est possible de réaliser le couplage magnétique d'autres manières en inversant par exemple l'agencement des parties magnétiques passives et actives respectivement dans le support de commande et le bouton stérilisable amovible, ou encore en agençant un nombre d'aimants permanents différents répartis entre chacune de ces deux pièces à assembler. Il est également possible d'employer des formes géométriques différentes pour le support de commande 20, ainsi que pour le bouton stérilisable 21 amovible, notamment en ce qui concerne les surfaces d'appui axiales et d'accouplement latérales. Similairement, le nombre de d'éléments prévus pour l'accrochage & l'entraînement en rotation (c'est-à-dire les languettes ou clips 26, les gorges latérales d'accouplement 29 pour le placement des O-rings 6, etc.) peuvent varier ainsi que leur disposition sur le support de commande 20 et respectivement le bouton stérilisable amovible 21 selon les besoins, pour autant que la force nécessaire pour l'arrachement reste dans une plage de valeur similaire à celle correspondant au mode de couplage magnétique. Il est également envisageable de combiner des caractéristiques relatives aux différentes modes de réalisation préférentiels et des différentes variantes décrits précédemment sans sortir du cadre de la présente invention.

## Revendications

1. Système de commande (1) pour micromoteur à usage dentaire ou chirurgical comprenant un boîtier (10) de commande pourvu d'une molette de réglage (2), montée rotative autour d'un axe de rotation (A-A), **caractérisé en ce que** ladite molette de réglage (2) est formée d'un support de commande (20) rotatif autour dudit axe de rotation (A-A) et solidaire d'un encodeur (3) intégré audit boîtier (10), et d'un bouton stérilisable (21) couplé de façon amovible audit support de commande (20), le couplage étant réalisé par friction, par clipsage, ou magnétiquement.

2. Système de commande (1) pour micromoteur à usage dentaire ou chirurgical selon l'une des revendications précédentes, **caractérisé en ce que** ladite molette de réglage (2) est prévue pour la sélection en rotation et l'actionnement par pression de fonctions, lesquelles sont visualisables sur un écran de contrôle (11) du boîtier (10).

3. Système de commande (1) pour micromoteur à usage dentaire ou chirurgical selon l'une des revendications précédentes, **caractérisé en ce que** ledit bouton stérilisable (21) amovible est terminé par une collerette (23) s'étendant radialement vers l'extérieur.

4. Système de commande (1) pour micromoteur à usage dentaire ou chirurgical selon l'une des revendications précédentes, **caractérisé en ce que** ledit bouton stérilisable (21) amovible est couplé audit support de commande (20) de manière à ce que, en position assemblée, un jeu de fonctionnement (E) d'au moins 0.2mm subsiste axialement entre l'extrémité inférieure dudit bouton stérilisable et la surface externe (12) du boîtier (10).

5. Système de commande (1) pour micromoteur à usage dentaire ou chirurgical selon l'une des revendications précédentes, **caractérisé en ce que** ledit bouton stérilisable (21) amovible présente une cavité centrale (23) pourvue d'au moins une première surface de contact intérieure (23a) pour l'accouplement à une surface d'appui (24), et **en ce que** des moyens de retenue axiaux et d'entraînement en rotation sont agencés de part et d'autre du bouton stérilisable (21) amovible et du support de commande (20).

6. Système de commande (1) pour micromoteur à usage dentaire ou chirurgical selon la revendication 5, **caractérisé en ce que** la cavité centrale (23) dudit bouton stérilisable (21) amovible débouche sur un épaulement périphérique intérieur (28).

7. Système de commande (1) pour micromoteur à usage dentaire ou chirurgical selon l'une des revendications précédentes, **caractérisé en ce que** ledit bouton stérilisable (21) amovible est couplé magnétiquement audit support de commande (20), une pièce magnétique active (4) étant intégrée dans ledit support de commande (20) et une pièce magnétique passive (5) étant intégrée dans ledit bouton stérilisable amovible (21), et l'intensité de la force magnétique exercée étant comprise entre 5 et 15 Newton.

8. Système de commande (1) pour micromoteur à usage dentaire ou chirurgical selon la revendication 7, lorsqu'elle dépend de la revendication 5, **caractérisé en ce que** la cavité centrale (23) du bouton stérilisable (21) amovible comprend une deuxième surface de contact intérieure (23b) conique, amenée en appui, en position assemblée de la molette de réglage (2), contre une surface d'accouplement latérale (25) dudit support de commande (20), lesdites surfaces de contact intérieure (23b) et d'accouplement latérale (25) présentant une inclinaison de 15 degrés par rapport à l'axe de rotation (A-A) de ladite molette de réglage (2).

9. Système de commande (1) pour micromoteur à usage dentaire ou chirurgical selon la revendication 7 ou 8, **caractérisé en ce que** ladite pièce magnétique active intégrée dans ledit support de commande (20) est un premier aimant permanent (4') agencé de manière à présenter une magnétisation orientée perpendiculairement par rapport à l'axe de rotation (A-A) de ladite molette de réglage (2), et qu'un deuxième aimant permanent (4") est intégré dans ledit bouton stérilisable amovible (21) de manière à présenter une magnétisation orientée selon une direction de magnétisation comprise dans un plan parallèle, superposé à celui du premier aimant permanent (4').

10. Système de commande (1) pour micromoteur à usage dentaire ou chirurgical selon la revendication 7 ou 8, **caractérisé en ce que** ladite pièce magnétique active intégrée dans ledit support de commande (20) est un premier aimant permanent (4') agencé de manière à présenter une magnétisation orientée selon l'axe de rotation (A-A) de ladite molette de réglage (2), et qu'un deuxième aimant permanent (4") est intégré dans ledit bouton stérilisable amovible (21) de manière à présenter une magnétisation orientée également selon l'axe de rotation (A-A) de ladite molette de réglage (2), mais dans le sens opposé par rapport à celui dudit premier aimant permanent (4').

11. Système de commande (1) pour micromoteur à usage dentaire ou chirurgical selon l'une des revendications précédentes, **caractérisé en ce que** ledit bouton stérilisable (21) amovible est couplé par friction au support de commande (20) grâce à des joints de type O-ring (6) insérés dans des gorges latérales d'accouplement (29).

12. Système de commande (1) pour micromoteur à usage dentaire ou chirurgical selon l'une des revendications précédentes, **caractérisé en ce que** ledit bouton stérilisable (21) amovible est assemblé au support de commande (20) à l'aide de clips (26) déformables élastiquement et pourvus d'ergots (26a) insérés dans des rainures d'accrochage (27).

## Patentansprüche

1. Steuersystem (1) für einen Mikromotor für einen zahnmedizinischen oder chirurgischen Einsatz, umfassend eine Steuerbox (10), die mit einem Einstelldrehknopf (2) versehen ist, welcher so angeordnet ist, dass er um eine Drehachse (A-A) drehbar ist, **dadurch gekennzeichnet, dass** der Einstelldrehknopf (2) aus einem Steuerträger (20), der um die Drehachse (A-A) drehbar und mit einem in der Steuerbox (2) integrierten Encoder (3) verbunden ist, und aus einem sterilisierbaren Knopf (21) geformt ist, der in einer abnehmbaren Weise an dem Steuerträger (20) verbunden ist, wobei die abnehmbare Verbindung durch Reibung, eine Schnappverbindung oder magnetisch erreicht wird.

2. Steuersystem (1) für einen Mikromotor für einen zahnmedizinischen oder chirurgischen Einsatz nach Anspruch 1, **dadurch gekennzeichnet, dass** der Einstelldrehknopf (2) zur Drehauswahl und Druckbestätigung von Funktionen vorgesehen ist, die auf einem Steuerbildschirm (11) der Box (10) sichtbar sind.

3. Steuersystem (1) für einen Mikromotor für einen zahnmedizinischen oder chirurgischen Einsatz nach einem der vorhergehenden Ansprüche, **dadurch gekennzeichnet, dass** der abnehmbare sterilisierbare Knopf (21) durch einen Kragen (22) begrenzt ist, der sich radial nach aussen erstreckt.

4. Steuersystem (1) für einen Mikromotor für einen zahnmedizinischen oder chirurgischen Einsatz nach einem der vorhergehenden Ansprüche, **dadurch gekennzeichnet, dass** der abnehmbare sterilisierbare Knopf (21) mit dem Steuerträger (20) derart gekoppelt ist, dass in montierter Position ein Betriebsspiel (E) von mindestens 0.2 mm axial zwischen dem unteren Ende des sterilisierbaren Knopfs und der Aussenfläche (12) der Box (10) besteht.

5. Steuersystem (1) für einen Mikromotor für einen zahnmedizinischen oder chirurgischen Einsatz nach einem der vorhergehenden Ansprüche, **dadurch gekennzeichnet, dass** der lösbare sterilisierbare Knopf (21) einen zentralen Hohlraum (23) aufweist, der mit mindestens einer ersten inneren Kontaktfläche (23a) zur Kopplung mit einer Stützfläche (24) versehen ist und dass Mittel zum axialen Zurückhalten und zum Drehantrieb zu beiden Seiten des abnehmbaren sterilisierbaren Knopfs (21) und dem Steuerträger (20) vorgesehen sind.

6. Steuersystem (1) für einen Mikromotor für einen zahnmedizinischen oder chirurgischen Einsatz nach einem der vorhergehenden Ansprüche, **dadurch gekennzeichnet** der zentrale Hohlraum (23) des abnehmbaren sterilisierbaren Knopfs (21) in eine innere Umfangsschulter (28) mündet.

7. Steuersystem (1) für einen Mikromotor für einen zahnmedizinischen oder chirurgischen Einsatz nach einem der vorhergehenden Ansprüche, **dadurch gekennzeichnet** der abnehmbare sterilisierbare Knopf (21) magnetisch mit dem Steuerträger (20) gekoppelt ist, ein aktives magnetisches Teil (4) in dem Steuerträger (20) integriert ist und ein passives magnetisches Teil (5) in dem abnehmbaren sterilisierbaren Knopf (21) integriert ist, und die Intensität der wirkenden magnetischen Kraft zwischen 5 und 15 Newton liegt.

8. Steuersystem (1) für einen Mikromotor für einen zahnmedizinischen oder chirurgischen Einsatz nach Anspruch 7, wenn abhängig von Anspruch 5, **dadurch gekennzeichnet, dass** der zentrale Hohlraum (23) des abnehmbaren sterilisierbaren Knopfs (21) eine zweite konische innere Kontaktfläche (23b) aufweist, die in der montierten Position des Einstelldrehknopfs (2) gegen eine laterale Kopplungsfläche (25) des Steuerträgers (20) in Anlage gebracht ist, wobei die innere Kontaktfläche (23b) und die laterale Kopplungsfläche (25) eine Neigung von 15° in Bezug auf die Drehachse (A-A) des Einstelldrehknopfs (2) aufweisen.

9. Steuersystem (1) für einen Mikromotor für einen zahnmedizinischen oder chirurgischen Einsatz nach Anspruch 7 oder 8, **dadurch gekennzeichnet, dass** das in dem Steuerträger (20) integrierte, aktive magnetische Teil ein erster Permanentmagnet (4') ist, der derart angeordnet ist, dass er eine Magnetisierung aufweist, die senkrecht zur Drehachse (A-A) des Einstelldrehknopfs (2) gerichtet ist, und dass ein zweiter Permanentmagneten (4") in dem abnehmbaren sterilisierbaren Knopf (21) integriert ist, um eine Magnetisierung zu haben, die in einer Magnetisierungsrichtung in einer parallelen Ebene orientiert ist, die der des ersten Permanentmagneten (4') überlagert ist.

10. Steuersystem (1) für einen Mikromotor für einen zahnmedizinischen oder chirurgischen Einsatz nach Anspruch 7 oder 8, **dadurch gekennzeichnet, dass** das in dem Steuerträger (20) integrierte aktive magnetische Teil ein erster Permanentmagnet (4') ist, der so angeordnet ist, um eine Magnetisierung zu haben, die entlang der Drehachse (A-A) des Einstelldrehknopfs (2) orientiert ist, und dass ein zweiter Permanentmagnet (4") in dem abnehmbaren sterilisierbaren Knopf (21) derart integriert ist, dass er eine Magnetisierung hat, welche ebenfalls entlang der Drehachse (A-A) des Einstelldrehknopfs (2) orientiert ist, aber in entgegengesetzter Richtung relativ zu derjenigen des ersten Permanentmagneten (4').

11. Steuersystem (1) für einen Mikromotor für einen zahnmedizinischen oder chirurgischen Einsatz nach einem der vorhergehenden Ansprüche, **dadurch gekennzeichnet, dass** der abnehmbare sterilisierbare Knopf (21) reibschlüssig mittels O-Ring Dichtungen (6), die in seitlichen Kupplungsnuten (29) eingefügt sind, mit dem Steuerträger (20) gekoppelt ist.

12. Steuersystem (1) für einen Mikromotor für einen zahnmedizinischen oder chirurgischen Einsatz nach einem der vorhergehenden Ansprüche, **dadurch gekennzeichnet, dass** der abnehmbare sterilisierbare Knopf (21) reibschlüssig mittels elastisch verformbarer Schnappverbindungen oder Zungen (26), die mit Haken (26a) versehen sind, welche Haken (26a) in Nuten (27) eingehakt sind, mit dem Steuerträger (20) verbunden ist.

## Claims

1. Control system (1) for a micro-motor for dental or surgical use comprising a control box (10) provided with an adjustment knob (2), mounted in a way rotatable about a rotational axis (A-A), **characterized in that** the said adjustment knob (2) is made up of a control support (20) rotatable about the said rotational axis (A-A) and integral with an encoder (3) integrated in the said box (10), and a sterilizable button (21) coupled in a removable way to the said control support (20), wherein the removable coupling is achieved through friction, through a snap-on connection, or magnetically.

2. Control system (1) for a micro-motor for dental or surgical use according claim 1, **characterized in that** the said adjustment knob (2) is provided for selecting in rotation and actuation by pressing of functions which are displayable on a control screen (11) of the box (10).

3. Control system (1) for a micro-motor for dental or surgical use according to one of the preceding claims, **characterized in that** the said removable sterilizable button (21) is terminated by a collar (22) extending radially outward.

4. Control system (1) for a micro-motor for dental or surgical use according to one of the preceding claims, **characterized in that** the said removable sterilizable button (21) is coupled to the said control support (20) in such a way that, in assembled position, an operational play (E) of at least 0.2 mm exists axially between the lower end of said sterilizable button and the outer surface (12) of the box (10).

5. Control system (1) for a micro-motor for dental or surgical use according to one of the preceding claims, **characterized in that** the said removable sterilizable button (21) has a central cavity (23) provided with at least one first inner contact surface (23a) for the coupling to a bearing surface (24), and **in that** means of axial retention and of driving in rotation are arranged on either side of the removable sterilizable button (21) and of the control support (20).

6. Control system (1) for a micro-motor for dental or surgical use according to claim 5, **characterized in that** the central cavity (23) of the said removable sterilizable button (21) comes out into an inner peripheral shoulder (28).

7. Control system (1) for a micro-motor for dental or surgical use according to one of the preceding claims, **characterized in that** the said removable sterilizable button (21) is coupled magnetically to the said control support (20), one active magnetic piece (4) being integrated in the said control support (20) and one passive magnetic piece (5) being integrated in the said removable sterilizable button (21), and the intensity of the exerted magnetic force being comprised within 5 and 15 Newton.

8. Control system (1) for a micro-motor for dental or surgical use according to claim 7, when it depends on claim 5, **characterized in that** the central cavity (23) of the removable sterilizable button (21) comprises a second conical inner contact surface (23b), brought to rest, in assembled position of the adjustment knob (2), against a lateral coupling surface (25) of the said control support (20), the said inner contact surface (23b) and lateral coupling surface (25) having an inclination of 15 degrees with respect to the axis of rotation (A-A) of the said adjustment knob (2).

9. Control system (1) for a micro-motor for dental or surgical use according to claim 7 or 8, **characterized in that** the said active magnetic piece integrated in the said control support (20) is a first permanent magnet (4') arranged in such a way as to have a magnetization oriented perpendicularly with respect to the axis of rotation (A-A) of the said adjustment knob (2), and that a second permanent magnet (4") is integrated in the said removable sterilizable button (21) in such a way as to have a magnetization oriented in a direction of magnetization encompassed in a parallel plane, overlying that of the first permanent magnet (4').

10. Control system (1) for a micro-motor for dental or surgical use according to claim 7 or 8, **characterized in that** the said active magnetic piece integrated in the said control support (20) is a first permanent magnet (4') arranged in such a way as to have a magnetization oriented along the axis of rotation (A-A) of the said adjustment knob (2), and that a second permanent magnet (4") is integrated in the said removable sterilizable button (21) in such a way as to have a magnetization likewise oriented along the axis of rotation (A-A) of the said adjustment knob (2), but in the opposite direction with respect to that of the said first permanent magnet (4').

11. Control system for a micro-motor for dental or surgical use according to any of the previous claims, wherein said removable sterilizable button (21) is coupled by friction to the control support (20) by means of O-ring type seals (6) inserted in lateral coupling recesses (29).

12. Control system for a micro-motor for dental or surgical use according to any of the previous claims, wherein said removable sterilizable button (21) is coupled by friction to the control support (20) by means with the aid of elastically deformable snap-on fasteners or tongues (26) provided with lugs (26a) inserted in hooking grooves (27).
